# EUROPÄISCHE PATENTSCHRIFT

(11) **EP 1 131 319 B1**
(45) Veröffentlichungstag und Bekanntmachung des Hinweises auf die Patenterteilung: **03.09.2003**
(21) Anmeldenummer: 99963288.8
(22) Anmeldetag: 06.11.1999
(51) Int. Cl.: C07D 413/04, C07D 417/04, C07D 413/14, C07D 417/14, C07D 239/54, A01N 43/76, A01N 43/78, C07C 275/30, C07C 271/22, C07C 271/54

(54) **HERBIZIDE 3-(BENZO(OX/THI)AZOL-7-YL)-1H-PYRIMIDIN-2,4-DIONE**
3- BENZ(OX/THI)AZOL-7-YL -1H-PYRIMIDINE-2,4-DIONES
3- BENZ(OX/THI)AZOL-7-YL]-1H-PYRIMIDIN-2,4-DIONES

(30) Priorität: 16.11.1998 DE 19852802
(43) Veröffentlichungstag der Anmeldung: 12.09.2001
(73) Patentinhaber: BASF AKTIENGESELLSCHAFT, 67056 Ludwigshafen (DE)
(72) Erfinder: REINHARD, Robert, D-67061 Ludwigshafen (DE); HAMPRECHT, Gerhard, D-69469 Weinheim (DE); SCHÄFER, Peter, D-67308 Ottersheim (DE); ZAGAR, Cyrill, D-67061 Ludwigshafen (DE); OTTEN, Martina, D-67069 Ludwigshafen (DE); WESTPHALEN, Karl-Otto, D-67346 Speyer (DE); WALTER, Helmut, D-67283 Obrigheim (DE); MENKE, Olaf, D-67317 Altleiningen (DE)
(74) Vertreter: Pohl, Michael, Dr.
(86) Internationale Anmeldenummer: EP9908514
(87) Internationale Veröffentlichungsnummer: WO00028822

(56) Entgegenhaltungen:
- EP-A- 0 255 047
- EP-A- 0 438 209
- EP-A- 0 869 123
- WO-A-95/17096
- WO-A-97/08170
- WO-A-97/08171
- WO-A-97/12884
- WO-A-98/33796
- WO-A-98/38188
- JP-A- 5 025 143
- US-A- 5 169 430

## Beschreibung

Die vorliegende Erfindung betrifft 3-[Benzoxazol-7-yl]-1H-pyrimidin-2,4-dione der Formel I 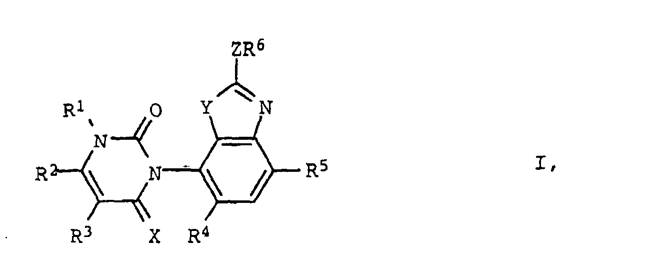 in der die Variablen folgende Bedeutungen haben:
- X: Sauerstoff oder Schwefel;
- Y: Sauerstoff;
- Z: eine chemische Bindung, C₁-C₄-Alkylen, Sauerstoff, Schwefel, SO oder SO₂;
- R¹: Wasserstoff, Amino, C₁-C₆-Alkyl oder C₁-C₆-Halogenalkyl;
- R²: Wasserstoff, Halogen, C₁-C₆-Alkyl, C₁-C₆-Halogenalkyl, C₁-C₆-Alkylthio, C₁-C₆-Alkylsulfinyl oder C₁-C₆-Alkylsulfonyl;
- R³: Wasserstoff, Halogen oder C₁-C₆-Alkyl;
- R⁴: Wasserstoff oder Halogen;
- R⁵: Cyano, Halogen, C₁-C₆-Alkyl, C₁-C₆-Halogenalkyl, C₁-C₆-Alkoxy oder C₁-C₆-Halogenalkoxy;
- R⁶: Wasserstoff, C₃-C₇-Cycloalkyl oder
3- bis 7-gliedriges gesättigtes Heterocyclyl, ein oder mehrere Sauerstoff und/oder Schwefel-Atome enthaltend,
wobei jeder Cycloalkyl- und jeder Heterocyclyl-Ring ein Carbonyl- oder Thiocarbonyl-Ringglied enthalten kann,
und wobei jeder Cycloalkyl- und Heterocyclyl-Ring unsubstituiert sein oder ein bis vier Substituenten tragen kann, jeweils ausgewählt aus der Gruppe bestehend aus Cyano, Nitro, Amino, Hydroxy, Halogen, C₁-C₄-Alkyl, C₁-C₄-Halogenalkyl, C₁-C₄-Cyanoalkyl, C₁-C₄-Hydroxyalkyl, C₁-C₄-Aminoalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfonyl, (C₁-C₄-Alkoxy)carbonyl, (C₁-C₄-Alkyl)carbonyl, (C₁-C₄-Halogenalkyl)carbonyl, (C₁-C₄-Alkyl)carbonyloxy, (C₁-C₄-Halogenalkyl)carbonyloxy, Di(C₁-C₄-alkyl)amino, C₃-C₆-Alkenyl, C₃-C₆-Alkinyl, C₃-C₄-Alkenyloxy, C₃-C₄-Alkenylthio, C₃-C₄-Alkinyloxy und C₃-C₄-Alkinylthio,
unter der Maßgabe, daß R⁶ nur dann für Wasserstoff steht, wenn Z eine chemische Bindung bedeutet
sowie die landwirtschaftlich brauchbaren Salze der Verbindungen I.

Außerdem betrifft die Erfindung
- die Verwendung der Verbindungen I als Herbizide,
- herbizide Mittel, welche die Verbindungen I als wirksame Substanzen enthalten,
- Verfahren zur Herstellung der Verbindungen I und von herbiziden Mitteln unter Verwendung der Verbindungen I,
- Verfahren zur Bekämpfung von unerwünschtem Pflanzenwuchs mit den Verbindungen I

In der WO 97/08170 werden bestimmte 3-(Benz(ox/thi)azol-7-yl)-6-(trifluormethyl)-uracile als Herbizide beschrieben. Weitere 3-(Benzthiazol-7-yl)uracile sowie deren Verwendung als Herbizide und zur Desikkation/Defoliation von Pflanzen werden in der WO 97/08171 gelehrt. Gegenstand der WO 97/12886 sind u.a. bestimmte 3-Benzisoxazol-7-yl-2,4-(1H,3H)pyrimidindione, denen eine herbizide und desikkante Wirkung zugeschrieben wird.

Aufgabe der vorliegenden Erfindung war es, neue herbizid wirksame Uracil-Verbindungen bereitzustellen, mit denen sich unerwünschte Pflanzen besser als mit den bekannten Uracil-Verbindungen gezielt bekämpfen lassen.

Demgemäß wurden die vorliegenden 3-[Benzoxazol-7-yl]-1H-pyrimidin-2,4-dione der Formel I gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Unter landwirtschaftlich brauchbaren Salzen kommen vor allem die Salze derjenigen Kationen oder die Säureadditionssalze derjenigen Säuren in Betracht, deren Kationen beziehungsweise Anionen die herbizide Wirkung der Verbindungen I nicht negativ beeinträchtigen. So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat. Sie können durch Reaktion von I mit einer Säure des entsprechenden Anions, vorzugsweise der Chlorwasserstoffsäure, Bromwasserstoffsäure, Schwefelsäure, Phosphorsäure oder Salpetersäure, gebildet werden.

Die bei der Definition der Substituenten R¹, R², R³, R⁵ und R⁶ oder als Reste an gesättigten Cycloalkyl- oder gesättigten heterocyclischen Ringen genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Cyanoalkyl-, Hydroxyalkyl-, Aminoalkyl-, Alkoxy-, Halogenalkoxy-, Alkylthio-, Halogenalkylthio-, Alkylsulfinyl-, Alkylsulfonyl-, Halogenalkylsulfonyl-, Alkenyl-, Alkenyloxy-, Alkenylthio-, Alkinyl-, Alkinyloxyund Alkinylthio- Teile können geradkettig oder verzweigt sein. Halogenierte Substituenten tragen vorzugsweise ein bis fünf gleiche oder verschiedene Halogenatome. Die Bedeutung Halogen steht jeweils für Fluor, Chlor, Brom oder Iod.

Ferner stehen beispielsweise:
- C₁-C₄-Alkyl für: Methyl, Ethyl, n-Propyl, iso-Propyl, n-Butyl, iso-Butyl, sek. Butyl oder tert. Butyl;
- C₁-C₄-Alkylen für: Methylen, 1,2-Ethylen, 1,1-Ethylen, 1,3-Propylen, 1,2-Propylen, 2,2-Propylen, 1,4-Butylen oder 2,3-Butylen;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. CH₂F, CHF₂, CF₃, CH₂Cl, CH(Cl)₂, C(Cl)₃, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, C₂F₅, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, CH₂-C₂F₅, CF₂-C₂F₅, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Alkyl für: einen C₁-C₄-Alkylrest wie vorstehend genannt, oder z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, vorzugsweise für CH₃, C₂H₅, CH₂-C₂H₅, CH(CH₃)₂, n-Butyl, C(CH₃)₃, n-Pentyl oder n-Hexyl;
- C₁-C₆-Halogenalkyl für: einen C₁-C₆-Alkylrest wie vorstehend genannt, der partiell oder vollständig durch Fluor, Chlor, Brom und/oder Iod substituiert ist, also z.B. einen der unter C₁-C₄-Halogenalkyl genannten Reste oder für 5-Fluor-1-pentyl, 5-Chlor-1-pentyl, 5-Brom-1-pentyl, 5-Iod-1-pentyl, 5,5,5-Trichlor-1-pentyl, Undecafluorpentyl, 6-Fluor-1-hexyl, 6-Chlor-1-hexyl, 6-Brom-1-hexyl, 6-Iod-1-hexyl, 6,6,6-Trichlor-1-hexyl oder Dodecafluorhexyl;
- Cyano-C₁-C₄-alkyl für: CH₂CN, 1-Cyanoethyl, 2-Cyanoethyl, 1-Cyanoprop-1-yl, 2-Cyanoprop-1-yl, 3-Cyanoprop-1-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 3-Cyanobut-2-yl, 4-Cyanobut-2-yl, 1-(CH₂CN)eth-1-yl, 1-(CH₂CN)-1-(CH₃)-eth-1-yl oder 1-(CH₂CN)prop-1-yl;
- Hydroxy-C₁-C₄-alkyl für: CH₂OH, 1-Hydroxyethyl, 2-Hydroxyethyl, 1-Hydroxyprop-1-yl, 2-Hydroxyprop-1-yl, 3-Hydroxyprop-1-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxybut-2-yl, 3-Hydroxybut-2-yl, 4-Hydroxybut-2-yl, 1-(CH₂OH)eth-1-yl, 1-(CH₂OH)-1-(CH₃)-eth-1-yl oder 1-(CH₂OH)prop-1-yl;
- Amino-C₁-C₄-alkyl für: CH₂NH₂, 1-Aminoethyl, 2-Aminoethyl, 1-Aminoprop-1-y 2-Aminoprop-1-yl, 3-Aminoprop-1-yl, 1-Aminobut-1-yl, 2-Aminobut-1-yl, 3-Aminobut-1-yl, 4-Aminobut-1-yl, 1-Aminobut-2-yl, 2-Aminobut-2-yl, 3-Aminobut-2-yl, 4-Aminobut-2-yl, 1-(CH₂NH₂)eth-1-yl, 1-(CH₂NH₂)-1-(CH₃)-eth-1-yl oder 1-(CH₂NH₂)prop-1-yl;
- C₁-C₄-Alkoxy für: OCH₃, OC₂H₅, OCH₂-C₂H₅, OCH(CH₃)₂, n-Butoxy, OCH(CH₃)-C₂H₅, OCH₂-CH(CH₃)₂ oder C(CH₃)₃, vorzugsweise für OCH₃, OC₂H₅ oder OCH(CH₃)₂;
- C₁-C₄-Halogenalkoxy für: einen C₁-C₄-Alkoxyrest wie vorstehend genannt, der partiell oder vollständig durch Fluor, Chlor, Brom und/oder Iod substituiert ist, also z.B. OCH₂F, OCHF₂, OCF₃, OCH₂Cl, OCH(Cl)₂, OC(Cl)₃, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy, vorzugsweise für OCHF₂, OCF₃, Dichlorfluormethoxy, Chlordifluormethoxy oder 2,2,2-Trifluorethoxy;
- C₁-C₆-Alkoxy für: einen C₁₋C₄₋Alkoxyrest wie vorstehend genannt, oder z.B. n-Pentoxy, 1-Methylbutoxy, 2-Methylbutoxy, 3-Methylbutoxy, 2,2-Dimethylpropoxy, 1-Ethylpropoxy, n-Hexoxy, 1,1-Dimethylpropoxy, 1,2-Dimethylpropoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy, vorzugsweise für OCH₃, OC₂H₅, OCH₂-C₂H₅, OCH(CH₃)₂, n-Butoxy, OC(CH₃)₃, n-Pentoxy oder n-Hexoxy;
- C₁-C₆-Halogenalkoxy für: einen C₁-C₆-Alkoxyrest wie vorstehend genannt, der partiell oder vollständig durch Fluor, Chlor, Brom und/oder Iod substituiert ist, also z.B. einen der unter C₁-C₄-Halogenalkoxy genannten Reste oder für 5-Fluor-1-pentoxy, 5-Chlor-1-pentoxy, 5-Brom-1-pentoxy, 5-Iod-1-pentoxy, 5,5,5-Trichlor-1-pentoxy, Undecafluorpentoxy, 6-Fluor-1-hexoxy, 6-Chlor-1-hexoxy, 6-Brom-1-hexoxy, 6-Iod-1-hexoxy, 6,6,6-Trichlor-1-hexoxy oder Dodecafluorhexoxy;
- C₁-C₄-Alkylthio für: SCH₃, SC₂H₅, SCH₂-C₂H₅, SCH(CH₃)₂, n-Butylthio, SCH(CH₃)-C₂H₅, SCH₂-CH(CH₃)₂ oder SC(CH₃)₃, vorzugsweise für SCH₃ oder SC₂H₅;
- C₁-C₄-Halogenalkylthio für: einen C₁-C₄-Alkylthiorest wie vorstehend genannt, der partiell oder vollständig durch Fluor, Chlor, Brom und/oder Iod substituiert ist, also z.B. SCH₂F, SCHF₂, SCF₃, SCH₂Cl, SCH(Cl)₂, SC(Cl)₃, Chlorfluormethylthio, Dichlorfluormethylthio, Chlordifluormethylthio, 2-Fluorethylthio, 2-Chlorethylthio, 2-Bromethylthio, 2-Iodethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio, SC₂F₅, 2-Fluorpropylthio, 3-Fluorpropylthio, 2,2-Difluorpropylthio, 2,3-Difluorpropylthio, 2-Chlorpropylthio, 3-Chlorpropylthio, 2,3-Dichlorpropylthio, 2-Brompropylthio, 3-Brompropylthio, 3,3,3-Trifluorpropylthio, 3,3,3-Trichlorpropylthio, SCH₂-C₂F₅, SCF₂-C₂F₅, 1-(CH₂F)-2-fluorethylthio, 1-(CH₂Cl)-2-chlorethylthio, 1-(CH₂Br)-2-bromethylthio, 4-Fluorbutylthio, 4-Chlorbutylthio, 4-Brombutylthio oder SCF₂-CF₂-C₂F₅, vorzugsweise für SCHF₂, SCF₃, Dichlorfluormethylthio, Chlordifluormethylthio oder 2,2,2-Trifluorethylthio;
- C₁-C₆-Alkylthio für: einen C₁₋C₄₋Alkylthiorest wie vorstehend genannt, oder z.B. n-Pentylthio, 1-Methylbutylthio, 2-Methylbutylthio, 3-Methylbutylthio, 2,2-Dimethylpropylthio, 1-Ethylpropylthio, n-Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio, vorzugsweise für SCH₃, SC₂H₅, SCH₂-C₂H₅, SCH(CH₃)₂, n-Butylthio, SC(CH₃)₃, n-Pentylthio oder n-Hexylthio;
- (C₁-C₄-Alkyl)carbonyl für: CO-CH₃, CO-C₂H₅, CO-CH₂-C₂H₅, CO-CH(CH₃)₂, n-Butylcarbonyl, CO-CH(CH₃)-C₂H₅, CO-CH₂-CH(CH₃)₂ oder CO-C(CH₃)₃, vorzugsweise für CO-CH₃ oder CO-C₂H₅;
- (C₁-C₄-Halogenalkyl)carbonyl für: einen (C₁-C₄-Alkyl)carbonylrest - wie vorstehend genannt - der partiell oder vollständig durch Fluor, Chlor, Brom und/oder Iod substituiert ist, also z.B. CO-CH₂F, CO-CHF₂, CO-CF₃, CO-CH₂Cl, CO-CH(Cl)₂, CO-C(Cl)₃, Chlorfluormethylcarbonyl, Dichlorfluormethylcarbonyl, Chlordifluormethylcarbonyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, CO-C₂F₅, 2-Fluorpropylcarbonyl, 3-Fluorpropylcarbonyl, 2,2-Difluorpropylcarbonyl, 2,3-Difluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, CO-CH₂-C₂F₅, CO-CF₂-C₂F₅, 1-(CH₂F)-2-fluorethylcarbonyl, 1-(CH₂Cl)-2-chlorethylcarbonyl, 1-(CH₂Br)-2-bromethylcarbonyl, 4-Fluorbutylcarbonyl, 4-Chlorbutylcarbonyl, 4-Brombutylcarbonyl oder CO-(n-C₄F₉), vorzugsweise für CO-CF₃, CO-CH₂Cl oder 2,2,2-Trifluorethylcarbonyl;
- (C₁-C₄-Alkyl)carbonyloxy für: O-CO-CH₃, O-CO-C₂H₅, O-CO-CH₂-C₂H₅, O-CO-CH(CH₃)₂, O-CO-CH₂-CH₂-C₂H₅, O-CO-CH(CH₃)-C₂H₅, O-CO-CH₂-CH(CH₃)₂ oder O-CO-C(CH₃)₃, vorzugsweise für O-CO-CH₃ oder O-CO-C₂H₅;
- (C₁-C₄-Halogenalkyl)carbonyloxy für: einen (C₁-C₄-Alkyl)carbonylrest - wie vorstehend genannt - der partiell oder vollständig durch Fluor, Chlor, Brom und/oder Iod substituiert ist, also z.B. O-CO-CH₂F, O-CO-CHF₂, O-CO-CF₃, O-CO-CH₂Cl, O-CO-CH(Cl)₂, O-CO-C(Cl)₃, Chlorfluormethylcarbonyloxy, Dichlorfluormethylcarbonyloxy, Chlordifluormethylcarbonyloxy, 2-Fluorethylcarbonyloxy, 2-Chlorethylcarbonyloxy, 2-Bromethylcarbonyloxy, 2-Iodethylcarbonyloxy, 2,2-Difluorethylcarbonyloxy, 2,2,2-Trifluorethylcarbonyloxy, 2-Chlor-2-fluorethylcarbonyloxy, 2-Chlor-2,2-difluorethylcarbonyloxy, 2,2-Dichlor-2-fluorethylcarbonyloxy, 2,2,2-Trichlorethylcarbonyloxy, O-CO-C₂F₅, 2-Fluorpropylcarbonyloxy, 3-Fluorpropylcarbonyloxy, 2,2-Difluorpropylcarbonyloxy, 2,3-Difluorpropylcarbonyloxy, 2-Chlorpropylcarbonyloxy, 3-Chlorpropylcarbonyloxy, 2,3-Dichlorpropylcarbonyloxy, 2-Brompropylcarbonyloxy, 3-Brompropylcarbonyloxy, 3,3,3-Trifluorpropylcarbonyloxy, 3,3,3-Trichlorpropylcarbonyloxy, O-CO-CH₂-C₂F₅, O-CO-CF₂-C₂F₅, 1-(CH₂F)-2-fluorethylcarbonyloxy, 1-(CH₂Cl)-2-chlorethylcarbonyloxy, 1-(CH₂Br)-2-bromethylcarbonyloxy, 4-Fluorbutylcarbonyloxy, 4-Chlorbutylcarbonyloxy, 4-Brombutylcarbonyloxy oder Nonafluorbutylcarbonyloxy, vorzugsweise für O-CO-CF₃, O-CO-CH₂Cl oder 2,2,2-Trifluorethylcarbonyloxy;
- (C₁-C₄-Alkoxy)carbonyl für: CO-OCH₃, CO-OC₂H₅, CO-OCH₂-C₂H₅, CO-OCH(CH₃)₂, n-Butoxycarbonyl, CO-OCH(CH₃)-C₂H₅, CO-OCH₂-CH(CH₃)₂ oder CO-OC(CH₃)₃, vorzugsweise für CO-OCH₃ oder CO-OC₂H₅;
- C₁-C₄-Alkylsulfinyl für: SO-CH₃, SO-C₂H₅, SO-CH₂-C₂H₅, SO-CH(CH₃)₂, SO-(n-C₄H₉), SO-CH(CH₃)-C₂H₅, SO-CH₂-CH(CH₃)₂ oder SO-C(CH₃)₃;
- C₁-C₆-Alkylsulfinyl für: einen C₁-C₄-Alkylsulfinylrest wie vorstehend genannt oder SO-(n-C₅H₁₁), 1-Methylbutyl-SO, 2-Methylbutyl-SO, 3-Methylbutyl-SO, 2,2-Dimethylpropyl-SO, 1-Ethylpropyl-SO, n-Hexyl-SO, 1,1-Dimethylpropyl-SO, 1,2-Dimethylpropyl-SO, 1-Methylpentyl-SO, 2-Methylpentyl-SO, 3-Methylpentyl-SO, 4-Methylpentyl-SO, 1,1-Dimethylbutyl-SO, 1,2-Dimethylbutyl-SO, 1,3-Dimethylbutyl-SO, 2,2-Dimethylbutyl-SO, 2,3-Dimethylbutyl-SO, 3,3-Dimethylbutyl-SO, 1-Ethylbutyl-SO, 2-Ethylbutyl-SO, 1,1,2-Trimethylpropyl-SO, 1,2,2-Trimethylpropyl-SO, 1-Ethyl-1-methylpropyl-SO oder 1-Ethyl-2-methylpropyl-SO, vorzugsweise für SO-CH₃, SO-C₂H₅, SO-CH₂-C₂H₅, SO-CH(CH₃)₂, SO-(n-C₄H₉), SO-C(CH₃)₃, SO-(n-C₅H₁₁) oder SO- (n-C₆H₁₃);
- C₁-C₄-Alkylsulfonyl für SO₂-CH₃, SO₂-C₂H₅, SO₂-CH₂-C₂H₅, SO₂-CH(CH₃)₂, SO₂-(n-C₄H₉), SO₂-CH(CH₃)-C₂H₅, SO₂-CH₂-CH(CH₃)₂ oder SO₂-C(CH₃)₃;
- C₁-C₆-Alkylsulfonyl für: einen C₁-C₄-Alkylsulfonylrest wie vorstehend genannt, oder SO₂₋(n-C₅H₁₁), 1-Methylbutyl-SO₂, 2-Methylbutyl-SO₂, 3-Methylbutyl-SO₂, 2,2-Dimethylpropyl-SO₂, 1-Ethylpropyl-SO₂, n-Hexyl-SO₂, 1,1-Dimethylpropyl-SO₂, 1,2-Dimethylpropyl-SO₂, 1-Methylpentyl-SO₂, 2-Methylpentyl-SO₂, 3-Methylpentyl-SO₂, 4-Methylpentyl-SO₂, 1,1-Dimethylbutyl-SO₂, 1,2-Dimethylbutyl-SO₂, 1,3-Dimethylbutyl-SO₂, 2,2-Dimethylbutyl-SO₂, 2,3-Dimethylbutyl-SO₂, 3,3-Dimethylbutyl-SO₂, 1-Ethylbutyl-SO₂, 2-Ethylbutyl-SO₂, 1,1,2-Trimethylpropyl-SO₂, 1,2,2-Trimethylpropyl-SO₂, 1-Ethyl-1-methylpropyl-SO₂ oder 1-Ethyl-2-methylpropyl-SO₂, vorzugsweise für SO₂-CH₃, SO₂-C₂H₅, SO₂-CH₂-C₂H₅, SO₂-CH(CH₃)₂, SO₂-(n-C₄H₉), SO₂-C(CH₃)₃, SO₂-(n-C₅H₁₁) oder SO₂-(n-C₆H₁₃) ;
- C₁-C₄-Halogenalkylsulfonyl für: einen C₁-C₄-Alkylsulfonylrestwie vorstehend genannt - der partiell oder vollständig durch Fluor, Chlor, Brom und/oder Iod substituiert ist, also z.B. SO₂-CH₂F, SO₂-CHF₂, SO₂-CF₃, SO₂-CH₂Cl, SO₂-CH(Cl)₂, SO₂-C(Cl)₃, Chlorfluormethylsulfonyl, Dichlorfluormethylsulfonyl, Chlordifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, SO₂₋C₂F₅, 2-Fluorpropylsulfonyl, 3-Fluorpropylsulfonyl, 2,2-Difluorpropylsulfonyl, 2,3-Difluorpropylsulfonyl, 2-Chlorpropylsulfonyl, 3-Chlorpropylsulfonyl, 2,3-Dichlorpropylsulfonyl, 2-Brompropylsulfonyl, 3-Brompropylsulfonyl, 3,3,3-Trifluorpropylsulfonyl, 3,3,3-Trichlorpropylsulfonyl, SO₂-CH₂-C₂F₅, SO₂-CF₂-C₂F₅, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl, vorzugsweise für SO₂-CH₂Cl, SO₂-CF₃ oder 2,2,2-Trifluorethylsulfonyl;
- Di(C₁-C₄-alkyl)amino für: N(CH₃)₂, N(C₂H₅)₂, N(CH₂-C₂H₅)₂, N[CH(CH₃)₂]₂, N,N-Dibutylamino, N,N-Di-(1-methylpropyl)amino, N,N-Di-(2-methylpropyl)amino, N[C(CH₃)₃]₂, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)-amino, N-(1-Methylpropyl)-N-(2-methylpropyl)amino, N-(1,1-Dimethylethyl)-N-(1-methylpropyl)amino oder N-(1,1-Dimethylethyl)-N-(2-methylpropyl)amino, vorzugsweise für N(CH₃)₂ oder N(C₂H₅)₂;
- C₃-C₆-Alkenyl für: Prop-1-en-1-yl, Allyl, 1-Methylethenyl, 1-Buten-1-yl, 1-Buten-2-yl, 1-Buten-3-yl, 2-Buten-1-yl, 1-Methyl-prop-1-en-1-yl, 2-Methyl-prop-1-en-1-yl, 1-Methylprop-2-en-1-yl, 2-Methyl-prop-2-en-1-yl, n-Penten-1-yl, n-Penten-2-yl, n-Penten-3-yl, n-Penten-4-yl, 1-Methylbut-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methylbut-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethylprop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methylpent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methylpent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethylbut-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethylbut-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₃-C₆-Alkinyl für: Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methylpent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise für Prop-2-in-1-yl;
- C₃-C₄-Alkenyloxy für: Allyloxy, But-1-en-3-yloxy, But-1-en-4-yloxy, But-2-en-1-yloxy, 1-Methylprop-2-enyloxy oder 2-Methylprop-2-enyloxy, vorzugsweise Allyloxy;
- C₃-C₄-Alkinyloxy für: Propargyloxy, But-1-in-3-yloxy, But-1-in-4-yloxy, But-2-in-1-yloxy, 1-Methylprop-2-inyloxy oder 2-Methylprop-2-inyloxy, vorzugsweise Propargyloxy;
- C₃-C₄-Alkinylthio für: Propargylthio, But-1-in-3-ylthio, But-1-in-4-ylthio, But-2-in-1-ylthio, 1-Methylprop-2-inylthio oder 2-Methylprop-2-inylthio, vorzugsweise Propargylthio;
- C₃-C₄-Alkenylthio für: Allylthlo, But-1-en-3-ylthio, But-1-en-4-ylthio, But-2-en-1-ylthio, 1-Methylprop-2-enylthio oder 2-Methylprop-2-enylthio, vorzugsweise Allylthio;
- C₃-C₇-Cycloalkyl für: Cyclopropyl, Cyclobutyl, Cyclopentyl, Cyclohexyl oder Cycloheptyl, vorzugsweise Cyclopropyl.

Unter 3- bis 7-gliedrigem gesättigtem Heterocyclylsystemen sind insbesondere solche mit
- ein oder zwei Sauerstoff- und/oder
- ein oder zwei Schwefelatomen
zu verstehen.

Beispiele für gesättigte Heterocyclen, die ein Carbonyl- oder Thiocarbonyl-Ringglied enthalten können, sind:
Oxiranyl, Thiiranyl, Oxetan-2-yl, Oxetan-3-yl, Thietan-2-yl, Thietan-3-yl, Tetrahydrofuran-2-yl, Tetrahydrofuran-3-yl, Tetrahydrothiophen-2-yl, Tetrahydrothiophen-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, 1,3-Dioxan-2-yl, 1,3-Dioxan-4-yl, 1,3-Dioxan-5-yl, 1,4-Dioxan-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, Oxepan-2-yl, Oxepan-3-yl, Oxepan-4-yl, Thiepan-2-yl, Thiepan-3-yl, Thiepan-4-yl, 1,3-Dioxepan-2-yl, 1,3-Dioxepan-4-yl, 1,3-Dioxepan-5-yl, 1,3-Dioxepan-6-yl, 1,3-Dithiepan-2-yl, 1,3-Dithiepan-4-yl, 1,3-Dithiepan-5-yl, 1,3-Dithiepan-6-yl, 1,4-Dioxepan-2-yl und 1,4-Dioxepan-7-yl.

Alle Cycloalkyl- und Heterocyclyl-Ringe sind vorzugsweise unsubstituiert oder tragen einen Substituenten.

Im Hinblick auf die Verwendung der erfindungsgemäßen 3-[Benzoxazol-7-yl]-1H-pyrimidin-2,4-dione I als Herbizide sind diejenigen Verbindungen I bevorzugt, bei denen die Variablen folgende Bedeutungen haben, und zwar jeweils für sich allein oder in Kombination:
- X: Sauerstoff;
- Y: Sauerstoff;
- Z: eine chemische Bindung, C₁-C₄-Alkylen, Sauerstoff oder Schwefel;
- R¹: Wasserstoff, Amino oder C₁-C₆-Alkyl;
- R²: Wasserstoff, Halogen, C₁-C₆-Alkyl, C₁-C₆-Halogenalkyl oder C₁-C₆-Alkylsulfonyl;
- R³: Wasserstoff:
- R⁴: Wasserstoff, Fluor oder Chlor;
- R⁵: Cyano oder Halogen;
- R⁶: Wasserstoff, C₃-C₆-Cycloalkyl oder
3- bis 7-gliedriges gesättigtes Heterocyclyl, ein Sauerstoffoder Schwefelatom enthaltend,
wobei jeder Cycloalkyl- und jeder Heterocyclyl-Ring ein Carbonyl- oder Thiocarbonyl-Ringglied enthalten kann,
und wobei jeder Cycloalkyl- und Heterocyclyl-Ring unsubstituiert sein oder ein bis vier Substituenten tragen kann, jeweils ausgewählt aus der Gruppe bestehend aus Cyano, Nitro, Amino, Hydroxy, Halogen, C₁-C₄-Alkyl, C₁-C₄-Halogenalkyl, C₁-C₄-Cyanoalkyl, C₁-C₄-Hydroxyalkyl, C₁-C₄-Aminoalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfonyl, (C₁-C₄-Alkoxy)carbonyl, (C₁-C₄-Alkyl)carbonyl, (C₁-C₄-Halogenalkyl)carbonyl, (C₁-C₄-Alkyl)carbonyloxy, (C₁-C₄-Halogenalkyl)carbonyloxy, Di(C₁-C₄-alkyl)amino, C₃-C₆-Alkenyl, C₃-C₆-Alkinyl, C₃-C₄-Alkenyloxy, C₃-C₄-Alkenylthio, C₃-C₄-Alkinyloxy und C₃-C₄-Alkinylthio;
- R⁶: insbesondere Wasserstoff, C₃-C₆-Cycloalkyl oder 3- bis 7-gliedriges gesättigtes Heterocyclyl, ein Sauerstoff- oder Schwefelatom enthaltend, wobei jeder Cycloalkyl- und jeder Heterocyclyl-Ring ein Carbonyl- oder Thiocarbonyl-Ringglied enthalten kann.

Ganz besonders bevorzugt sind die 3-[Benzoxazol-7-yl]-1H-pyrimidin-2,4-dione der Formel Ia {≙ I mit X und Y = Sauerstoff, R¹ = Methyl, R² = Trifluormethyl, R³ = Wasserstoff, R⁴ = Fluor, R⁵ = Chlor und Z = chemische Bindung} 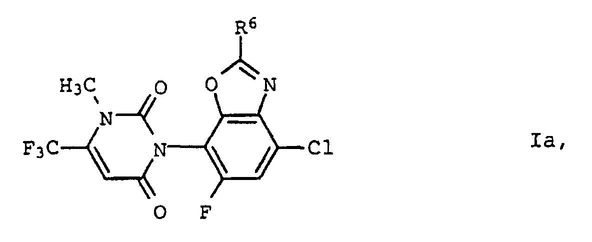 insbesondere die in der folgenden Tabelle 1 aufgeführten Verbindungen Ia.1 bis Ia.51:

**Tabelle 1**

| **Nr.** | **R**^{**6**} |
|---|---|
| Ia.1 | Cyclopropyl |
| Ia.2 | Cyclobutyl |
| Ia.3 | Cyclopentyl |
| Ia.4 | Cyclohexyl |
| Ia.5 | Cycloheptyl |
| Ia.6 | Oxiran-2-yl |
| Ia.7 | Oxetan-2-yl |
| Ia.8 | Tetrahydrofuran-2-yl |
| Ia.9 | Tetrahydropyran-2-yl |
| Ia.10 | Oxepan-2-yl |
| Ia.11 | Thiiran-2-yl |
| Ia.12 | Thietan-2-yl |
| Ia.13 | Tetrahydrothiofuran-2-yl |
| Ia.14 | Tetrahydrothiopyran-2-yl |
| Ia.15 | Thiepan-2-yl |
| Ia.16 | Oxetan-3-yl |
| Ia.17 | Tetrahydrofuran-3-yl |
| Ia.18 | Tetrahydropyran-3-yl |
| Ia.19 | Oxepan-3-yl |
| Ia.20 | Thietan-3-yl |
| Ia.21 | Tetrahydrothiofuran-3-yl |
| Ia.22 | Tetrahydrothiopyran-3-yl |
| Ia.23 | Thiepan-3-yl- |
| Ia.24 | Tetrahydropyran-4-yl |
| Ia.25 | Oxepan-4-yl |
| Ia.26 | Tetrahydrothiopyran-4-yl |
| Ia.27 | Thiepan-4-yl |
| Ia.28 | 2-Oxocyclopropyl |
| Ia.29 | 2-Oxocyclobutyl |
| Ia.30 | 2-Oxocyclopentyl |
| Ia.31 | 2-Oxocyclohexyl |
| Ia.32 | 2-Oxocycloheptyl |
| Ia.33 | 2-Thioxo-cyclopropyl |
| Ia.34 | 2-Thioxo-cyclobutyl |
| Ia.35 | 2-Oxo-oxetan-3-yl |
| Ia.36 | 2-Oxo-tetrahydrofuran-3-yl |
| Ia.37 | 2-Oxo-tetrahydropyran-3-yl |
| Ia.38 | 2-Oxo-oxepan-3-yl |
| Ia.39 | 2-Thioxo-thietan-3-yl |
| Ia.40 | 2-Thioxo-tetrahydrothien-3-yl |
| Ia.41 | 2-Thioxo-tetrahydrothiopyran-3-yl |
| Ia.42 | 2-Thioxo-thiepan-3-yl |
| Ia.43 | 2-Thioxo-oxetan-3-yl |
| Ia.44 | 2-Thioxo-tetrahydrofuran-3-yl |
| Ia.45 | 2-Thioxo-tetrahydropyran-3-yl |
| Ia.46 | 2-Thioxo-oxepan-3-yl |
| Ia.47 | 2-Oxo-thietan-3-yl |
| Ia.48 | 2-Oxo-tetrahydrothien-3-yl |
| Ia.49 | 2-Oxo-tetrahydrothiopyran-3-yl |
| Ia.50 | 2-Oxo-thiepan-3-yl |
| Ia.51 | H |

Des weiteren sind die 3-[Benzoxazol-7-yl]-1H-pyrimidin-2,4-dione der Formeln Ib bis Ip besonders bevorzugt, insbesondere
- die Verbindungen Ib.1 - Ib.51, die sich von den entsprechenden Verbindungen Ia.1 - Ia.51 lediglich dadurch unterscheiden, daß R¹ für Wasserstoff steht: 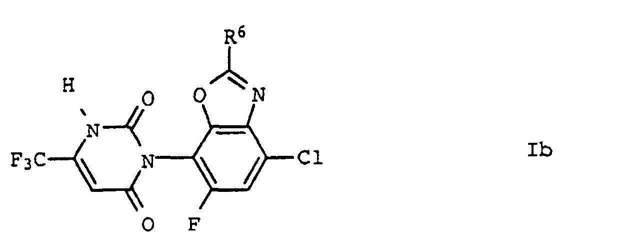
- die Verbindungen Ie.1 - Ie.51, die sich von den entsprechenden Verbindungen Ia.1 - Ia.51 lediglich dadurch unterscheiden, daß Z für CH₂ steht: 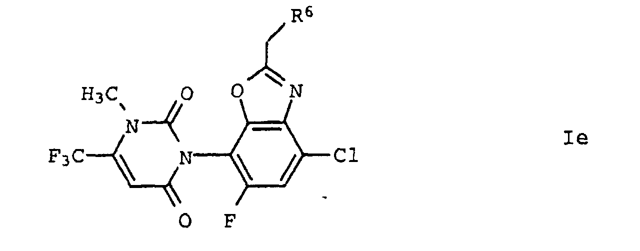
- die Verbindungen If.1 - If.51, die sich von den entsprechenden Verbindungen Ia.1 - Ia.51 lediglich dadurch unterscheiden, daß R¹ für Wasserstoff und Z für CH₂ stehen: 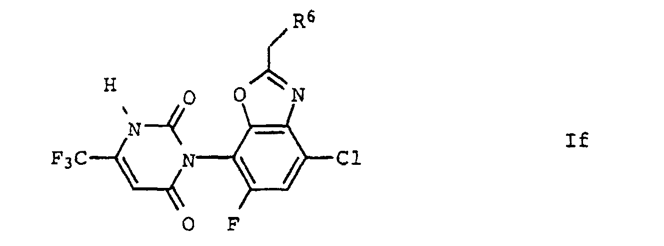
- die Verbindungen Ii.1 - Ii.51, die sich von den entsprechenden Verbindungen Ia.1 - Ia.51 lediglich dadurch unterscheiden, daß Z für Sauerstoff steht: 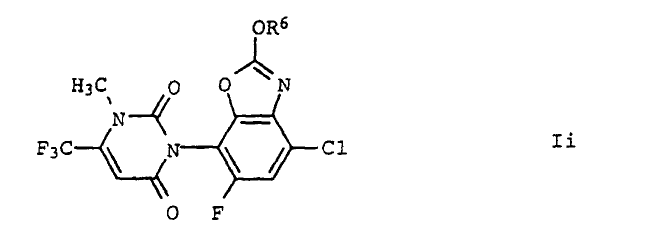
- die Verbindungen Ij.1 - Ij.51, die sich von den entsprechenden Verbindungen Ia.1 - Ia.51 lediglich dadurch unterscheiden, daß R¹ für Wasserstoff und Z für Sauerstoff stehen: 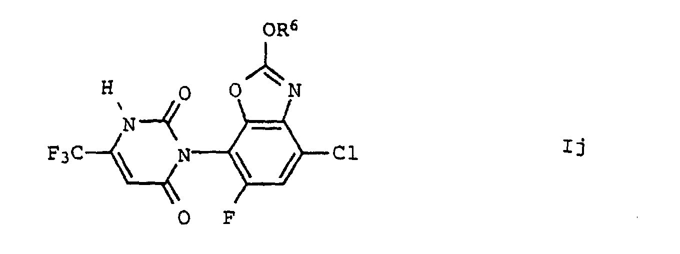
- die Verbindungen Im.1 - Im.51, die sich von den entsprechenden Verbindungen Ia.1 - Ia.51 lediglich dadurch unterscheiden, daß Z für Schwefel steht: 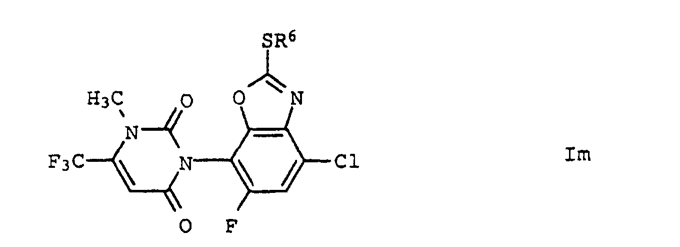
- die Verbindungen In.1 - In.51, die sich von den entsprechenden Verbindungen Ia.1 - Ia.51 lediglich dadurch unterscheiden, daß R¹ für Wasserstoff und Z für Schwefel stehen:

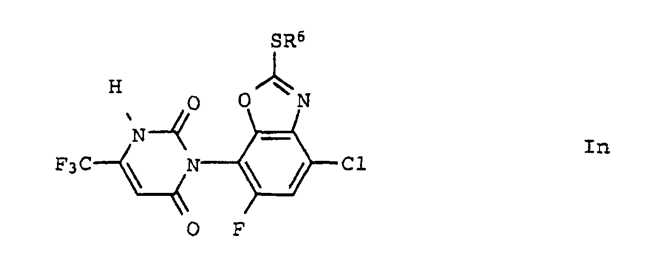

Die erfindungsgemäßen
3-[Benzoxazol-7-yl]-1H-pyrimidin-2,4-dione der Formel I sind auf verschiedene Weise erhältlich, beispielsweise nach einem der folgenden Verfahren:

### Verfahren A)

Kondensation eines substituierten 2-Aminophenols oder 2-Aminothiophenols mit Kohlensäure- oder Carbonsäurederivaten:

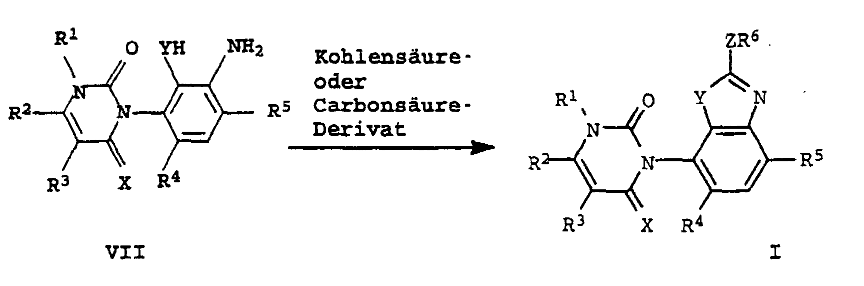

Die Kondensationsreaktion von bifunktionellen Benzolen VII mit Kohlensäure- oder Carbonsäurederivaten kann auf an sich bekannte Weise durchgeführt werden (vgl. z.B. Houben-Weyl, Methoden der organischen Chemie, Georg Thieme Verlag Stuttgart, Bd. E8c, 1. Auflage 1994, S. 247-284; Bd. E8b, 1. Auflage 1994, S. 881-901; Bd. E8a, 1. Auflage 1993, S. 1032-1078). Bevorzugte Kohlensäure- oder Carbonsäurederivate sind die entsprechenden Ester, Anhydride, Säurechloride, Orthoester, Diimide, Nitrile, Imidoester, Trichlormethyl-substituierte Verbindungen, Isocyanate und deren Thioanaloga.

Als Lösungs-/Verdünnungsmittel kommen insbesondere organische Lösungsmittel in Betracht, beispielsweise aromatische Kohlenwasserstoffe wie Benzol, Toluol und o-, m-, p-Xylol, halogenierte Kohlenwasserstoffe wie Methylenchlorid, Chloroform und Dichlorethan, niedere Alkohole wie Methanol und Ethanol, aliphatische oder cyclische Ether wie Dimethoxyethan, Tetrahydrofuran und Dioxan, Carbonsäureester wie Essigsäureethylester oder aprotisch polare Solvention wie Dimethylformamid und Dimethylsulfoxid.

Die Reaktion kann gewünschtenfalls durch Zusatz katalytischer Mengen einer Säure beschleunigt werden. Als Säuren eignen sich insbesondere Mineralsäuren wie Salzsäuren oder Sulfonsäuren wie p-Toluolsulfonsäure bzw. deren Salze mit Stickstoffbasen wie Pyridin. Die Mengen an Säure liegen bevorzugt bei 0,01 bis 5 Molprozenten, bezogen auf die Menge an VII.

Die Reaktionstemperaturen liegen bevorzugt bei 20°C bis Rückflußtemperatur des jeweiligen Reaktionsgemisches, insbesondere bei 60°C bis Rückflußtemperatur.

Das Kohlensäure- oder Carbonsäurederivat wird entweder in ca. stöchiometrischer Menge oder im Überschuß angewandt. In geeigneten Fällen kann auch ein sehr großer Überschuß eingesetzt oder ohne Lösungsmittel gearbeitet werden. Bevorzugt sind etwa stöchiometische Mengen oder ein Überschuß von bis zu 10 Moläquivalenten, bezogen auf die Menge an VII.

Die substituierten 2-Aminophenole und -thiophenole erhält man zweckmäßigerweise durch Reduktion entsprechender 2-Nitrophenole oder -thiophenole VIII (vgl. z.B. Houben-Weyl, Methoden der organischen Chemie, Georg Thieme Verlag Stuttgart, Bd. XI/1, 4. Auflage 1957, S. 431ff.):

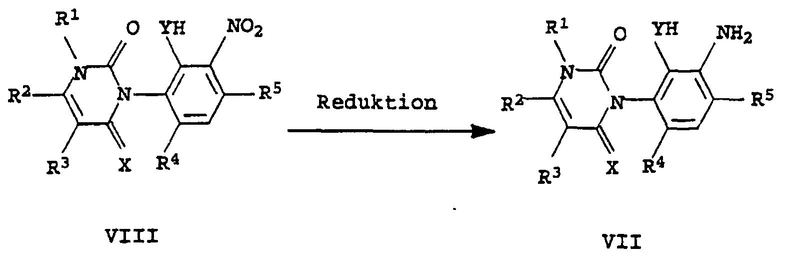

Als Reduktionsmittel kommen insbesondere
- elementare Metalle wie Eisen, Zinn und Zink,
- Wasserstoff in Gegenwart von geeigneten Katalysatoren wie Palladium oder Platin auf Kohle oder Raney-Nickel, oder
- komplexe Hydride wie LiAlH₄ und NaBH₄, ggf. in Gegenwart von Katalysatoren,
in Betracht.

Als Lösungsmittel eignen sich üblicherweise - je nach Reduktionsmittel - Carbonsäuren wie Essigsäure und Propionsäure, Mineralsäuren wie Salzsäure und Schwefelsäure, Alkohole wie Methanol und Ethanol, Ether wie Diethylether, Methyl-tert.-butylether, Tetrahydrofuran und Dioxan, Aromaten wie Benzol und Toluol, sowie Gemische derartiger Solventien.

Die Umsetzungen können bei Temperaturen von (-100)°C bis zur Siedetemperatur des jeweiligen Reaktionsgemisches vorgenommen werden.

Üblicherweise werden die Ausgangsverbindungen in etwa stöchiometrischen Mengen eingesetzt; in Einzelfällen kann jedoch auch ein vielfacher Überschuß der einen oder anderen Komponente vorteilhaft sein.

Die 2-Nitrophenole oder -thiophenole VIII können durch Umsetzung von Vorstufen IX erhalten werden, bei denen am Phenol-Sauerstoff bzw. Thiophenol-Schwefel-Atom verschiedene Gruppen gebunden sind. Es können dies z.B. Alkyl-, Benzyl-, Alkylcarbonyl-, Arylcarbonyl- oder Alkoxycarbonyl-Gruppen (R) sein.

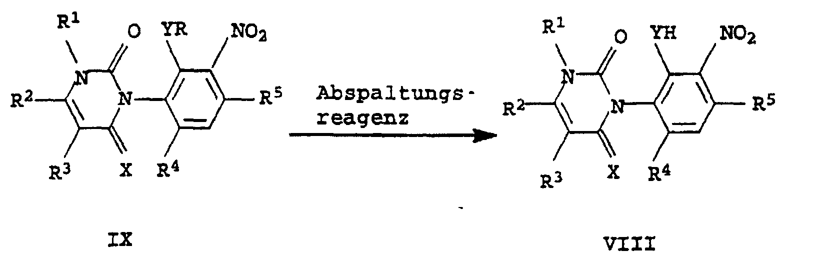

Als Abspaltungsreagenzien kommen insbesondere in Betracht:
- bei gegebenenfalls substituierten Alkylphenolen: Trimethylsilyliodid, Bortribromid, Bortrichlorid, Aluminiumtrichlorid, Lithiumchlorid oder Bromwasserstoff;
- bei gegebenenfalls substituierten Benzylphenolen oder -thiophenolen: Bortrifluorid, Fluorwasserstoffsäure oder Wasserstoff/Katalysator, dabei bevorzugt Edelmetall-Katalysatoren wie Palladium und Platin;
- bei gegebenenfalls substituierten Arylestern oder -thioestern: Natriumhydrogencarbonat, Natriumcarbonat, Kaliumcarbonat, Natriumhydroxid, Kaliumhydroxid, Natriummethylat, Chlorwasserstoff, Schwefelsäure, Ammoniak, Hydrazin oder Zink.

Das Lösungs-/Verdünnungsmittel ist bevorzugt so zu wählen, daß es gegenüber dem jeweiligen Abspaltungsreagenz inert ist. Bei Verwendung der Halogenide Trimethylsilyliodid, Bortribromid, Bortrichlorid oder Aluminiumtrichlorid sind halogenierte Lösungsmittel wie Dichlormethan, Chloroform, Tetrachlorkohlenstoff und Dichlorethan besonders bevorzugt. Bromwasserstoff wird bevorzugt in wäßriger Lösung angewandt, ganz besonders bevorzugt als eine 48 gew.%ige Lösung; Lithiumchlorid wird bevorzugt in polaren Lösungsmitteln wie niederen Alkoholen, Dimethylsulfoxid und Dimethylformamid angewendet; hydrogenolytische Methoden werden bevorzugt in niederen Alkoholen oder Carbonsäuren durchgeführt, gegebenenfalls unter Zusatz eines Wasserstoffüberträgers wie Cyclohexen und Cyclohexadien. Mineralbasen bzw. Mineralsäuren und Stickstoffbasen werden bevorzugt im wässrigen Medium angewendet. Je nach Löslichkeit der Reagenzien wird gegebenenfalls ein organisches Lösungsmittel, z.B. ein niederer Alkohol, zugesetzt.

Die Temperatur für die Abspaltungsreaktion liegt bevorzugt bei 0°C bis Siedetemperatur des jeweiligen Reaktionsgemisches.

Das Abspaltungsreagenz wird bevorzugt in etwa stöchiometrischen Mengen oder im Überschuß angewendet. Der Überschuß liegt besonders bevorzugt zwischen einem und zehn Moläquivalenten, bezogen auf die Menge an IX.

### Verfahren B)

Nukleophiler Austausch von Fluor in Nitroaromaten VI durch eine Sauerstoff- bzw. Schwefelfunktion:

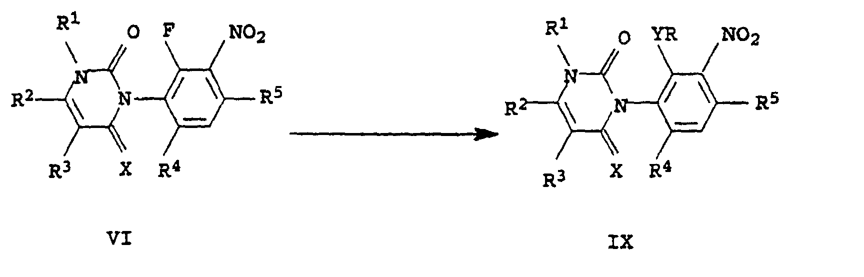

Die Austauschreaktion von aromatisch gebundenem Fluor durch Sauerstoff bzw. Schwefelnukleophile wird auf an sich bekannte Weise durchgeführt (vgl. z.B. Houben-Weyl, Methoden der organischen Chemie, Georg Thieme Verlag Stuttgart, 1976, Bd. 6/1c, S. 146-202 und Band IX, 4. Auflage 1955, S. 7-18).

Folgende Reagenzien sind bei dieser Reaktion bevorzugte Reaktionspartner:
- zum Austausch F gegen OR: Kaliumbenzoat, Natriumnitrit, Natriumhydroxid, Kaliumhydroxid, Kaliumcarbonat, Benzaldoxim, Natriumacetat, Kaliumacetat, Natriummethylat, Kaliummethylat, Natriumtrimethylsilanolat, Äpfelsäuredimethylester;
- zum Austausch F gegen SR: Natriumsulfid, Natriumhydrogensulfid, Kaliumhydrogensulfid, Benzylmercaptan.

Die Reaktionen können in bestimmten Fällen ohne Lösungsmittel/Verdünnungsmittel durchgeführt werden. Bei Verwendung eines Lösungs-/Verdünnungsmittels sind solche zu bevorzugen, in denen das Sauerstoff- bzw. Schwefelgruppen-übertragende Reagenz eine gute Löslichkeit besitzt. Besonders bevorzugt sind Alkohole wie Ethanol, Propanol und tert.-Butanol, aprotisch polare Lösungsmittel wie Dimethylformamid und Dimethylacetamid, cyclische Ether wie Dioxan und Tetrahydrofuran oder aliphatische Ether wie Dimethoxyethan.

Die Reaktionstemperaturen liegen bevorzugt bei 20°C bis Rückflußtemperatur des jeweiligen Reaktionsgemisches, insbesondere bei 60°C bis Rückflußtemperatur.

Das Sauerstoff- bzw. Schwefelgruppen-übertragende Reagenz wird entweder in ca. stöchiometrischer Menge oder im Überschuß angewandt. In geeigneten Fällen kann auch ein sehr großer Überschuß eingesetzt werden. Bevorzugt sind etwa stöchiometische Mengen oder ein Überschuß von bis zu 10 Moläquivalenten, bezogen auf die Menge an VI.

### Verfahren C)

Umsetzung eines 3-Phenylpyrimidindion-Derivats I, bei dem R¹ Wasserstoff bedeutet, mit einer alkylierenden Verbindung II auf an sich bekannte Weise: 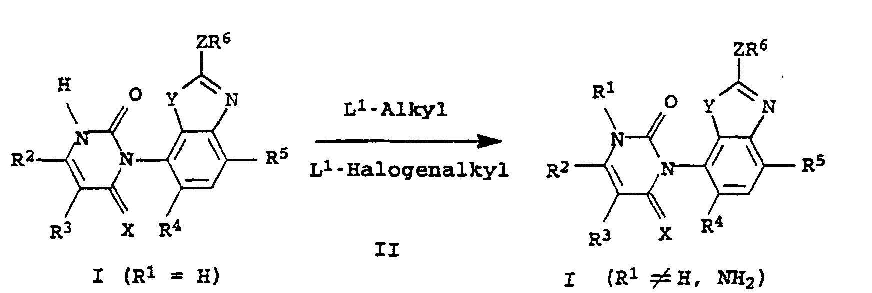 L¹ steht für eine übliche Abgangsgruppe wie Halogen, vorzugsweise Chlor, Brom oder Iod, (Halogen)alkylsulfonyloxy, vorzugsweise Methylsulfonyloxy oder Trifluormethylsulfonyloxy, Arylsulfonyloxy, vorzugsweise Toluolsulfonyloxy, und Alkoxysulfonyloxy, vorzugsweise Methoxysulfonyloxy oder Ethoxysulfonyloxy.

Üblicherweise arbeitet man in einem inerten organischen Lösungsmittel, beispielsweise in einem protischen Lösungsmittel wie den niederen Alkoholen, vorzugsweise in Methanol oder Ethanol, gewünschtenfalls im Gemisch mit Wasser, oder in einem aprotischen Lösungsmittel, z.B. in einem aliphatischen oder cyclischen Ether wie Methyl-tert.-butylether, 1,2-Dimethoxyethan, Tetrahydrofuran und Dioxan, in einem aliphatischen Keton wie Aceton, Diethylketon und Ethylmethylketon, in einem Amid wie Dimethylformamid und N-Methylpyrrolidon, in einem Sulfoxid wie Dimethylsulfoxid, in einem Harnstoff wie Tetramethylharnstoff und 1,3-Dimethyltetrahydro-2(1H)-pyrimidinon, in einem Carbonsäureester wie Essigsäureethylester, oder in einem halogenierten aliphatischen oder aromatischen Kohlenwasserstoff wie Dichlormethan, Dichlorethan, Chlorbenzol und den Dichlorbenzolen.

Gewünschtenfalls kann in Gegenwart einer Base gearbeitet werden, wobei sowohl anorganische Basen, z.B. Carbonate wie Natriumcarbonat und Kaliumcarbonat, Hydrogencarbonate wie Natrium- und Kaliumhydrogencarbonat, oder Alkalimetallhydride wie Natriumhydrid und Kaliumhydrid, als auch organische Basen, z.B. Amine wie Triethylamin, Pyridin und N,N-Diethylanilin, oder Alkalimetallalkoholate wie Natriummethanolat, Natriumethanolat und Kaliumtert.-butanolat, geeignet sind.

Die Menge an Base und Alkylierungsmittel II liegt vorzugsweise jeweils bei der 0,5- bis 2-fachen molaren Menge, bezogen auf die Menge an Ausgangsverbindung I (mit R¹ = Wasserstoff).

Im allgemeinen liegt die Reaktionstemperatur bei 0°C bis zur Siedetemperatur des Reaktionsgemisches, insbesondere bei 0 bis 60°C.

Besonders vorteilhaft ist es, das Natriumsalz durch Auflösen des Phenylpyrimidindion-Derivats I mit R¹ = Wasserstoff in einer wäßrigen Natriumhydroxid-Lösung bei 20 bis 25°C herzustellen, wobei etwa äquivalente Mengen an Phenylpyrimidindion-Derivat I (mit R¹ = H) und Natriumhydroxid eingesetzt werden. Das entsprechende Salz des Phenylpyrimidindion-Derivats I kann dann z.B. durch Fällen mit einem geeigneten inerten Lösungsmittel oder durch Abdampfen des Lösungsmittels isoliert werden.

Salze der Phenylpyrimidindion-Derivate I, deren Metallion kein Alkalimetallion ist, können üblicherweise durch Umsalzen des entsprechenden Alkalimetallsalzes in wäßriger Lösung hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

### Verfahren D)

Umsetzung eines Phenylpyrimidindion-Derivats der Formel I, wobei R¹ Wasserstoff bedeutet, mit einem elektrophilen Aminierungsreagenz in Gegenwart einer Base:

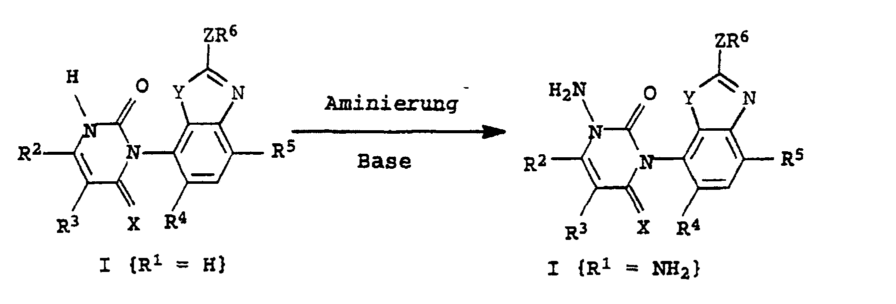

Als Aminierungsreagenz hat sich bisher 2,4-Dinitrophenoxyamin besonders bewährt, jedoch kann z.B. auch Hydroxylamin-O-sulfonsäure (HOSA) verwendet werden, die aus der Literatur bereits als Aminierungsreagenz bekannt ist (vgl. z.B. E. Hofer et al., Synthesis 1983, 466; W. Friedrichsen et al., Heterocycles 20 (1983) 1271; H. Hart et al., Tetrahedron Lett. 25 (1984) 2073; B. Vercek et al., Monatsh. Chem. 114 (1983) 789; G. Sosnousky et al., Z. Naturforsch. 38 (1983) 884; R.S. Atkinson et al., J. Chem. Soc. Perkin Trans. 1987, 2787).

Die Aminierung kann auf an sich bekannte Weise durchgeführt werden (siehe z.B. T. Sheradsky, Tetrahedron Lett. 1968, 1909; M.P. Wentland et al., J. Med. Chem. 27 (1984) 1103 und insbesondere EP-A 240 194, EP-A 476 697 und EP-A 517 181, wo die Aminierung von Uracilen gelehrt wird).

Normalerweise führt man die Umsetzung in einem polaren Lösungsmittel durch, z.B. in Dimethylformamid, N-Methylpyrrolidon, Dimethylsulfoxid oder in Ethylacetat, das sich bisher als besonders geeignet erwiesen hat.

Als Base eignen sich beispielsweise Alkalimetallcarbonate wie Kaliumcarbonat, Alkalimetallalkoholate wie Natriummethylat und Kalium-tert.-butanolat oder Alkalimetallhydride wie Natriumhydrid.

Die Menge an Base und Aminierungsmittel liegt vorzugsweise jeweils bei der 0,5- bis 2-fachen molaren Menge, bezogen auf die Menge an Ausgangsverbindung.

### Verfahren E)

Schwefelung eines 3-(Benzazol-7-yl)pyrimidindion-Derivats der Formel I, bei dem X Sauerstoff bedeutet:

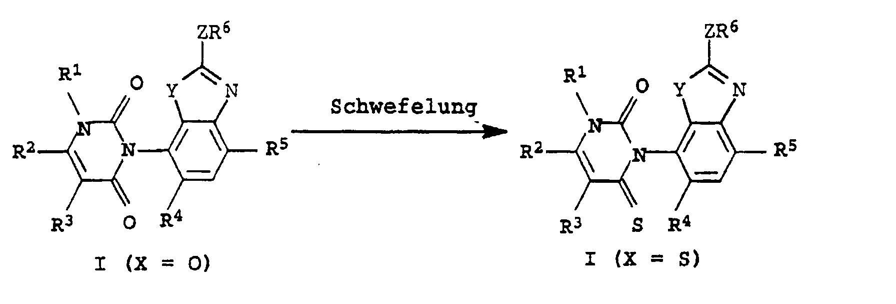

Die Schwefelung erfolgt in der Regel in einem inerten Lösungs- oder Verdünnungsmittel, beispielsweise in einem aromatischen Kohlenwasserstoff wie Toluol und den Xylolen, in einem Ether wie Diethylether, 1,2-Dimethoxyethan und Tetrahydrofuran, oder in einem organischen Amin wie Pyridin.

Als Schwefelungsreagenz eignen sich besonders gut Phosphor(V)-sulfid und 2,4-Bis(4-methoxyphenyl)-1,3,2,4-dithiadiphosphetan-2,4-dithion ("Lawesson-Reagenz").

Üblicherweise ist die 1- bis 5-fache molare Menge, bezogen auf die zu schwefelnde Ausgangsverbindung, für eine weitgehend vollständige Umsetzung ausreichend.

Die Reaktionstemperatur liegt normalerweise bei 20 bis 200°C, vorzugsweise bei 40°C bis zur Siedetemperatur des Reaktionsgemisches.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen. Im allgemeinen setzt man die Reaktionspartner in einem Molverhältnis von 0,95:1 bis 5:1 ein.

Die Aufarbeitung der Reaktionsgemische erfolgt in der Regel nach an sich bekannten Methoden, beispielsweise durch Verdünnen der Reaktionslösung mit Wasser und anschließender Isolierung des Produktes mittels Filtration, Kristallisation oder Lösungsmittelextraktion, oder durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die Verbindungen I und deren landwirtschaftlich brauchbaren Salze eignen sich - sowohl als lsomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N. rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (S. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel 5 können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die 3-(Benzazol-7-yl)pyrimidindion-Derivate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Liginin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die erfindungsgemäßen Verbindungen I können beispielsweise wie folgt formuliert werden:
- I: 20 Gewichtsteile einer Verbindung I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
- II: 20 Gewichtsteile einer Verbindung I werden in einer Mischung gelöst, die aus 40 Gewichtsteilen Cyclohexanon, 30 Gewichtsteilen lsobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
- III: 20 Gewichtsteile eines Wirkstoffs I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
- IV: 20 Gewichtsteile eines Wirkstoffs I werden mit 3 Gewichtsteilen des Natriumsalzes der DiiSobutylnaphthalinsulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.
- V: 3 Gewichtsteile eines Wirkstoffs I werden mit 97 Gewichtsteilen feinteiligem Kaolin vermischt. Man erhält auf diese weise ein Stäubemittel, das 3 Gew.% des Wirkstoffs enthält.
- VI: 20 Gewichtsteile eines Wirkstoffs I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII: 1 Gewichtsteil einer Verbindung I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII: 1 Gewichtsteil einer Verbindung I wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die erfindungsgemäßen [Benz(ox/ thi)azol-7-yl]-1H-pyrimidin-2,4-dione mit zahlreichen Vertretern anderer herbizider oder wachstumsregulierender Wirkstoffgruppen gemischt und gemeinsam ausgebracht werden. Beispielsweise kommen als Mischungspartner 1 ,2,4-Thiadiazole, 1 ,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1 ,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.)

### Herstellungsbeispiele

### Beispiel 1

### 3-[4-Chlor-2-cyclopentyl-6-fluorbenzoxazol-7-yl]-1-methyl-6-trifluormethyl-2,4-(1H,3H)-pyrimidindion (Verb. Ia.3 aus Tabelle 1)

0,48 g 3-[3-Amino-4-chlor-6-fluor-2-hydroxyphenyl]-1-methyl-6-trifluormethyl-2,4-(1H,3H)-pyrimidindion (aus Vorstufe 7) wurden mit 0,14 g Triethylamin in 30 ml absolutem Xylol vorgelegt und bei 0°C tropfenweise mit 0,18 g Cyclopentancarbonsäurechlorid versetzt. Nach 10 Minuten wurde auf 20°C erwärmt, 0,1 g Pyridinium-p-toluolsulfonat zugesetzt und die Mischung sechs Stunden auf Rückfluß erhitzt. Nach Abkühlen wurde die Xylolphase dreimal mit 30 ml Wasser gewaschen, über Natriumsulfat getrocknet und im Vakuum vom Lösungsmittel befreit. Die Reinigung des Rohprodukts erfolgte mittels Mitteldruckflüssigkeitschromatographie (MPLC); Eluent: Cyclohexan/Ethylacetat (9:1). Ausbeute: 0,27 g;
¹H-NMR (200 MHz, in CDCl₃): δ [ppm] = 7.25 (d, 1H), 6.40 (s, 1H), 3.60 (s, 3H), 3.30 (quin, 1H), 1.50-2.25 (m, 8H).

### Vorstufe 1

### 4-Chlor-2,6-difluoranilin

35,5 g 2,6-Difluoranilin wurden mit 200 ml konzentrierter Essigsäure vermischt und auf 80°C erwärmt. 42,4 g Sulfurylchlorid wurden mit etwa der gleichen Menge konzentrierter Essigsäure vermischt und zu der erwärmten Lösung zugetropft. Danach wurde die Mischung für sechs Stunden auf Rückfluß erhitzt. Nach dem Abkühlen der Lösung wurde am Vakuum eingeengt und der Rückstand mit Wasser und Pentan verrührt. Der feste Rückstand wurde abfiltriert und mit Wasser gewaschen. Der so erhaltene Feststoff wurde mit 200 ml konzentrierter Salzsäure versetzt und für zwei Stunden auf Rückfluß erhitzt. Nach dem Abkühlen wurde die Lösung vorsichtig mit Natronlauge schwach alkalisch gestellt und dreimal mit 100 ml Ethylacetat extrahiert. Die organische Phase wurde mit Natriumsulfat getrocknet und vom Lösungsmittel befreit. Ausbeute: 40 g; ¹H-NMR (270 MHz, in CDCl₃) : δ [ppm] = 6.85 (d, 2H), 3.5-3.9 (br, 2H).

### Vorstufe 2

### 4-Chlor-2,6-difluorphenylisocyanat

40 g 4-Chlor-2,6-difluoranilin wurden in 300 ml absolutem Toluol gelöst. Unter Rühren wurden 100 ml Chlorameisensäuretrichlormethylester bei 20°C zugetropft. Die Mischung wurde langsam auf Rückfluß erhitzt. Nach dreieinhalb Stunden wurde die Mischung eingeengt und das so erhaltene Isocyanat direkt weiter für die Synthese von Vorstufe 3 verwendet.

### Vorstufe 3

### 3-[4-Chlor-2,6-difluorphenyl]-6-trifluormethyl-2,4-(1H,3H)-pyrimidindion

7,2 g Natriumhydrid wurden in 300 ml absolutem Dimethylformamid vorgelegt und 44 g 3-Amino-4,4,4-trifluorbut-2-ensäureethylester (gelöst in wenig Dimethylformamid) wurden bei 0°C zugetropft. Zwei Stunden wurde die Mischung bei dieser Temperatur gerührt. Die Mischung wurde auf (-20)°C gekühlt und das Isocyanat aus Vorstufe 2 wurde in wenig absolutem Tetrahydrofuran zugetropft. Nach beendeter Zugabe wurde auf 20°C aufgetaut und die Mischung für 18 Stunden gerührt. Danach wurden die flüchtigen Bestandteile im Vakuum entfernt; nun wurde mit verdünnter Salzsäure versetzt und mit Methyl-tert.-butylether (dreimal 100 ml) extrahiert. Die organische Phase wurde mit Natriumsulfat getrocknet und vom Lösungsmittel befreit. Ausbeute: 72,0 g; ¹H-NMR (270 MHz, in CDCl₃): δ [ppm] = 7.10 (d, 2H), 6.25 (s, 1H).

### Vorstufe 4

### 3-[4-Chlor-2,6-difluorphenyl]-1-methyl-6-trifluormethyl-2,4-(1H,3H)-pyrimidindion

71 g 3-[4-Chlor-2,6-difluorphenyl]-6-trifluormethyl-2,4-(1H,3H)-pyrimidindion wurden in 200 ml absolutem Dimethylformamid vorgelegt, 29,0 g Kaliumcarbonat wurden bei 20°C zugegeben, dann tropfenweise 29,8 g Methyliodid. Die Mischung wurde 18 Stunden gerührt. Das Lösungsmittel wurde im Vakuum entfernt, der Rückstand mit Wasser versetzt und mit Methyl-tert.-butylether extrahiert. Nach Trocknen über Natriumsulfat wurde das Lösungsmittel im Vakuum entfernt. Ausbeute: 58,0 g; ¹H-NMR (270 MHz, in CDCl₃): δ [ppm] = 7.10 (d, 2H), 6.40 (s, 1H), 3.55 (s, 3H).

### Vorstufe 5

### 3-[4-Chlor-2,6-difluor-3-nitrophenyl]-1-methyl-6-trifluormethyl-2,4-(1H,3H)-pyrimidindion

58 g 3-[4-Chlor-2,6-difluorphenyl]-1-methyl-6-trifluormethyl-2,4-(1H,3H)-pyrimidindion wurden in 400 ml konzentrierter Schwefelsäure gelöst und bei 0°C mit einer Mischung aus 10,7 g 98 %iger Salpetersäure und 10 ml konzentrierter Schwefelsäure tropfenweise versetzt. Dreißig Minuten wurde bei 0°C nachgerührt, danach 18 Stunden bei 20°C. Anschließend wurden nochmals 10,7 g der Säuremischung bei 0°C zugetropft. Nach zwei Stunden Rühren bei 20°C wurde auf Eiswasser gegossen, der dabei erhaltene Niederschlag abgetrennt und mit Wasser neutral gewaschen. Der Feststoff wurde im Vakuumtrockenschrank getrocknet. Ausbeute: 58,0 g;
¹H-NMR (270 MHz, in CDCl₃): δ [ppm] = 7.30 (d, 1H), 6.40 (s, 1H), 3.60 (s, 3H)

### Vorstufe 6

### 3-[4-Chlor-6-fluor-2-hydroxy-3-nitrophenyl]-1-methyl-6-trifluormethyl-2,4-(1H,3H)-pyrimidindion

30 g 3-[4-Chlor-2,6-difluor-3-nitrophenyl]-1-methyl-6-trifluormethyl-2,4-(1H,3H)-pyrimidindion wurden in 100 ml absolutem Dimethylformamid gelöst, mit 25,2 g Natriumacetat versetzt und vier Stunden auf 100°C erhitzt. Nach dem Abkühlen wurde das Lösungsmittel im Vakuum entfernt und der Rückstand mit Wasser versetzt; durch Zugabe von verdünnter Salzsäure wird der pH in den sauren Bereich gebracht und mit Methyl-tert.-butylether (dreimal 75 ml) extrahiert. Man erhielt das Wertprodukt im Gemisch mit dem durch Austausch des zur Nitrogruppe para-ständigen Fluoratoms entstehenden Isomeren. Gesamtausbeute: 28,3 g; Das Rohprodukt wurde auf der Folgestufe (Vorstufe 7) gereinigt.
¹H-NMR (270 MHz, in CDCl₃): δ [ppm] = 7.05 (d, 1H), 6.40 (s, 1H), 3.55 (s, 3H).

### Vorstufe 7

### 3-[3-Amino-4-chlor-6-fluor-2-hydroxyphenyl]-1-methyl-6-trifluormethyl-2,4-(1H,3H)-pyrimidindion

28 g Rohprodukt aus Vorstufe 6 wurden in einem Gemisch aus 260 ml Wasser, 50 ml Ethanol und 26,7 ml konzentrierter Salzsäure gelöst. Die Mischung wurde auf 65°C erwärmt und 20,7 g Eisenpulver wurden portionsweise zugegeben. Die Mischung wurde sechs Stunden auf Rückfluß erhitzt. Nach Abkühlen wurde mit Ethylacetat (300 ml) extrahiert. Das nach Entfernen des Lösungsmittels im Vakuum erhaltene Rohprodukt wurde per Säulenchromatographie (Eluent: Cyclohexan/Ethylacetat = 8:2) gereinigt. Ausbeute: 2,8 g; ¹H-NMR (270 MHz, in CDCl₃): δ [ppm] = 6.80 (d, 1H), 6.30 (s, 1H), 4.6-5.2 (br, 2H), 3.50 (s, 3H).

In der nachfolgenden Tabelle 2 sind neben den vorstehend beschriebenen noch weitere 3-[Benzoxazol-7-yl]-1H-pyrimidin-2,4-dione der Formel I aufgeführt, die auf analoge weise hergestellt wurden oder herstellbar sind:

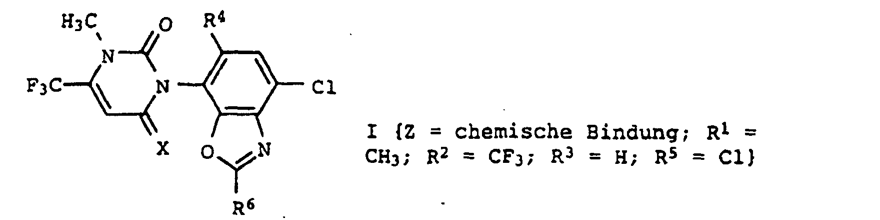

**Tabelle 2:**

| Bsp.-Nr. | X | R⁴ | R⁶ | Fp. [°C] |
|---|---|---|---|---|
| 1 | O | F | Cyclopentyl | Öl |
| 2 | O | F | Cyclohexyl | Öl |
| 3 | O | F | Tetrahydropyran-3-yl | Öl |
| 4 | O | F | H | 185 |
| 5 | O | F | Cyclopropyl | Öl |
| 6 | O | F | Tetrahydropyran-4-yl | Öl |
| 7 | O | F | Tetrahydrothiopyran-4-yl | Öl |
| 8 | O | F | Cyclobutyl | Öl |
| 9 | S | F | Cyclobutyl | Öl |
| 10 | O | H | Cyclopentyl m | Öl |
| 11 | O | H | Cyclopentyl | Öl |

Die herbizide Wirkung der 3-[Benzoxazol-7-yl]-1H-pyrimidin-2,4-dione der Formel I ließ sich durch Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmaßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug lediglich 15,6 bzw. 7,8 g/ha a. S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | | |
|---|---|---|
| Lateinischer Name | Deutscher Name | Englischer Name |
| Abutilon theophrasti | Chinesischer Hanf | velvet Ieaf |
| Amaranthus retroflexus | zurückgekrümmter Fuchsschwanz | redroot pigweed |
| Chenopodium album | Weißer Gänseruß | lambsquarters |
| Solanum nigrum | Schwarzer Nachtschatten | black nightshade |

Bei Aufwandmengen von nur 15,6 bzw. 7,8 g/ha bewirkte Verbindung Nr. Ia.3 eine völlige Zerstörung der 4 oben aufgeführten Testpflanzen (Wirkungsgrad 100 %).

## Patentansprüche

1. 3-[Benzoxazol-7-yl]-1H-pyrimidin-2,4-dione der Formel I 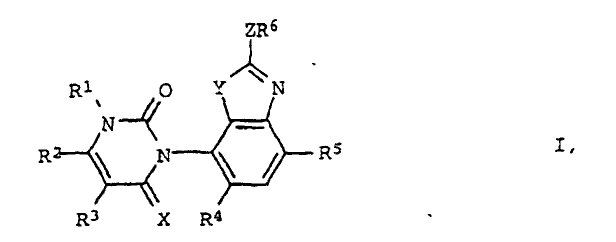 in der die Variablen folgende Bedeutungen haben:
X Sauerstoff oder Schwefel;
Y Sauerstoff ;
Z eine chemische Bindung, C₁-C₄-Alkylen, Sauerstoff, Schwefel, SO oder SO₂;
R¹ Wasserstoff, Amino, C₁-C₆-Alkyl oder C₁-C₆-Halogenalkyl;
R² Wasserstoff, Halogen, C₁-C₆-Alkyl, C₁-C₆-Halogenalkyl, C₁-C₆-Alkylthio, C₁-C₆-Alkylsulfinyl oder C₁-C₆-Alkylsulfonyl;
R³ Wasserstoff, Halogen oder C₁-C₆-Alkyl;
R⁴ Wasserstoff oder Halogen;
R⁵ Cyano, Halogen, C₁-C₆-Alkyl, C₁-C₆-Halogenalkyl, C₁-C₆-Alkoxy oder C₁-C₆-Halogenalkoxy;
R⁶ Wasserstoff, C₃-C₇-Cycloalkyl oder 3- bis 7-gliedriges gesättigtes Heterocyclyl, ein oder mehrere Sauerstoff und/oder Schwefel-Atome enthaltend, wobei jeder Cycloalkyl- und jeder Heterocyclyl-Ring ein Carbonyl- oder Thiocarbonyl-Ringglied enthalten kann,
und wobei jeder Cycloalkyl- und Heterocyclyl-Ring unsubstituiert sein oder ein bis vier Substituenten tragen kann, jeweils ausgewählt aus der Gruppe bestehend aus Cyano, Nitro, Amino, Hydroxy, Halogen, C₁-C₄-Alkyl, C₁-C₄-Halogenalkyl, C₁-C₄-Cyanoalkyl, C₁-C₄-Hydroxyalkyl, C₁-C₄-Aminoalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfonyl, (C₁-C₄-Alkoxy)carbonyl, (C₁-C₄-Alkyl)carbonyl, (C₁-C₄-Halogenalkyl)carbonyl, (C₁-C₄-Alkyl)carbonyloxy, (C₁-C₄-Halogenalkyl)carbonyloxy, Di(C₁-C₄-alkyl)amino, C₃-C₆-Alkenyl, C₃-C₆-Alkinyl, C₃-C₄-Alkenyloxy, C₃-C₄-Alkenylthio, C₃-C₄-Alkinyloxy und C₃-C₄-Alkinylthio,
unter der Maßgabe, daß R⁶ nur dann für Wasserstoff steht, wenn Z eine chemische Bindung bedeutet,
sowie die landwirtschaftlich brauchbaren Salze der Verbindungen I.

2. 3-[Benzoxazol-7-yl]-1H-pyrimidin-2,4-dione der Formel I nach Anspruch 1, wobei
X für Sauerstoff,
Z für eine chemische Bindung, C₁-C₄-Alkylen, Sauerstoff oder Schwefel,
R¹ für Wasserstoff, Amino oder C₁-C₆-Alkyl,
R² für Wasserstoff, Halogen, C₁-C₆-Alkyl, C₁-C₆-Halogenalkyl oder C₁-C₆-Alkylsulfonyl,
R³ für Wasserstoff,
R⁴ für Wasserstoff, Fluor oder Chlor und
R⁵ für Cyano oder Halogen stehen.

3. Verwendung der 3-[Benzoxazol-7-yl)-1H-pyrimidin-2,4-dione I und ihrer landwirtschaftlich brauchbaren Salze, gemäß Anspruch 1, als Herbizide.

4. Herbizides Mittel, enthaltend eine herbizid wirksame Menge mindestens eines 3-[Benzoxazol-7-yl]-1H-pyrimidin-2,4-dions der Formel I oder eines landwirtschaftlich brauchbaren Salzes von I, gemäß Anspruch 1, und mindestens einen flüssigen und/oder festen Trägerstoff sowie gewünschtenfalls mindestens einen oberflächenaktiven Stoff.

5. Verfahren zur Herstellung von herbizid wirksamen Mitteln, **dadurch gekennzeichnet, daß** man eine herbizid wirksame Menge mindestens eines 3- [Benzoxazol-7-yl]-1H-pyrimidin-2,4-dions der Formel I oder eines landwirtschaftlich brauchbaren Salzes von I, gemäß Anspruch 1, mit mindestens einem inerten flüssigen und/oder festen Trägerstoff sowie gewünschtenfalls mindestens einem oberflächenaktiven Stoff mischt.

6. Verfahren zur Bekämpfung von unerwünschtem Pflanzenwuchs, **dadurch gekennzeichnet, daß** man eine herbizid wirksame Menge mindestens eines 3-[Benzoxazol-7-yl]-1H-pyrimidin-2,4-dions der Formel I oder eines landwirtschaftlich brauchbaren Salzes von I, gemäß Anspruch 1, auf Pflanzen, deren Lebensraum oder auf Saatgut einwirken läßt.

7. Verfahren zur Herstellung von 3-[Benzoxazol-7-yl]-1H-pyrimidin-2,4-dionen I gemäß Anspruch 1, **dadurch gekennzeichnet, daß** man Aminophenole bzw. Aminothiophenole der Formel VII 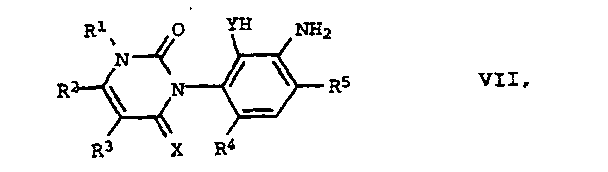 wobei die Variablen X und R¹ bis R⁵ die in Anspruch 1 angegebenen Bedeutungen haben,
mit Carbonsäure- bzw. Kohlensäurederivaten kondensiert.

## Claims

1. A 3-[benzoxazol-7-yl]-1H-pyrimidine-2,4-dione of the formula I 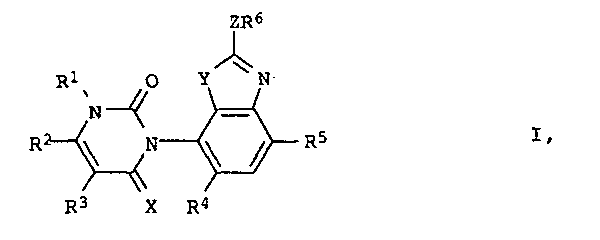 in which:
X is oxygen or sulfur;
Y is oxygen;
Z is a chemical bond, C₁-C₄-alkylene, oxygen, sulfur, SO or SO₂;
R¹ is hydrogen, amino, C₁-C₆-alkyl or C₁-C₆-haloalkyl;
R² is hydrogen, halogen, C₁-C₆-alkyl, C₁-C₆-haloalkyl, C₁-C₆-alkylthio, C₁-C₆-alkylsulfinyl or C₁-C₆-alkylsulfonyl;
R³ is hydrogen, halogen or C₁-C₆-alkyl;
R⁴ is hydrogen or halogen;
R⁵ is cyano, halogen, C₁-C₆-alkyl, C₁-C₆-haloalkyl, C₁-C₆-alkoxy or C₁-C₆-haloalkoxy;
R⁶ is hydrogen, C₃-C₇-cycloalkyl or 3- to 7-membered saturated heterocyclyl containing one or more oxygen and/or sulfur atoms, where each cycloalkyl and each heterocyclyl ring may contain a carbonyl or thiocarbonyl ring member,
and where each cycloalkyl and heterocyclyl ring may be unsubstituted or may carry from one to four substituents, in each case selected from the group consisting of cyano, nitro, amino, hydroxyl, halogen, C₁-C₄-alkyl, C₁-C₄-haloalkyl, C₁-C₄-cyanoalkyl, C₁-C₄-hydroxyalkyl, C₁-C₄-aminoalkyl, C₁-C₄-alkoxy, C₁-C₄-haloalkoxy, C₁-C₄-alkylthio, C₁-C₄-haloalkylthio, C₁-C₄-alkylsulfinyl, C₁-C₄-alkylsulfonyl, C₁-C₄-haloalkylsulfonyl, (C₁-C₄-alkoxy)carbonyl, (C₁-C₄-alkyl)carbonyl, (C₁-C₄-haloalkyl)carbonyl, (C₁-C₄-alkyl)carbonyloxy, (C₁-C₄-haloalkyl)carbonyloxy, di(C₁-C₄-alkyl)amino, C₃-C₆-alkenyl, C₃-C₆-alkynyl, C₃-C₄-alkenyloxy, C₃-C₄-alkenylthio, C₃-C₄-alkynyloxy and C₃-C₄-alkynylthio,
with the proviso that R⁶ is hydrogen only if Z is a chemical bond.
or an agriculturally useful salt of a compound I.

2. A 3-[benzoxazol-7-yl]-1H-pyrimidine-2,4-dione of the formula I as claimed in claim 1 where
X is oxygen,
Z is a chemical bond, C₁-C₄-alkylene, oxygen or sulfur,
R¹ is hydrogen, amino or C₁-C₆-alkyl,
R² is hydrogen, halogen, C₁-C₆-alkyl, C₁-C₆-haloalkyl or C₁-C₆-alkylsulfonyl,
R³ is hydrogen,
R⁴ is hydrogen, fluorine or chlorine and
R⁵ is cyano or halogen.

3. The use of a 3-[benzoxazol-7-yl]-lH-pyrimidine- 2,4-dione I or its agriculturally useful salt as claimed in claim 1 as a herbicide.

4. A herbicidal composition, comprising a herbicidally effective amount of at least one 3-[benzoxazol-7-yl)-1H- pyrimidine-2,4-dione of the formula I or an agriculturally useful salt of I as claimed in claim 1 and at least one liquid and/or solid carrier and, if desired, at least one surfactant.

5. A process for preparing herbicidal compositions, which comprises mixing a herbicidally effective amount of at least one 3-[benzoxazol-7-yl]-1H-pyrimidine-2,4-dione of the formula I or an agriculturally useful salt of I as claimed in claim 1 with at least one inert liquid and/or solid carrier and, if desired, at least one surfactant.

6. A method for controlling undesirable vegetation, which comprises allowing a herbicidally effective amount of at least one 3-[benzoxazol-7-yl]-1H-pyrimidine-2,4-dione of the formula I or an agriculturally useful salt of I as claimed in claim 1 to act on plants, their habitat or on seeds.

7. A process for preparing a 3-[benzoxazol-7-yl]-1H- pyrimidine-2,4-dione as claimed in claim 1, which comprises condensing aminophenols or aminothiophenols of the formula VII 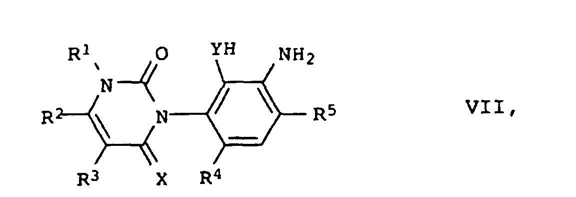 where the variables X and R¹ to R⁵ are each as defined in claim 1,
with carboxylic acid derivatives or carbonic acid derivatives.

## Revendications

1. 3-[benzoxazol-7-yl]-1H-pyrimidine-2,4-diones répondant à la formule I 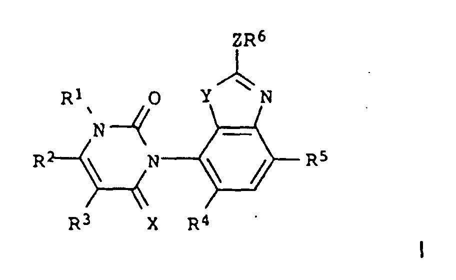 dans laquelle les variables ont les significations ci-après :
X représente un atome d'oxygène ou un atome de soufre ;
Y représente un atome d'oxygène ;
Z représente une liaison chimique, un groupe alkylène en C₁ - C₄, un atome d'oxygène, un atome de soufre, un groupe SO ou un groupe SO₂ ;
R¹ représente un atome d'hydrogène, un groupe amino, un groupe alkyle en C₁ - C₆ ou un groupe halogénalkyle en C₁ - C₆ ;
R² représente un atome d'hydrogène, un atome d'halogène, un groupe alkyle en C₁ - C₆, un groupe halogénalkyle en C₁ - C₆, un groupe alkyl(en C₁ - C₆)thio, un groupe alkyl(en C₁ - C₆)sulfinyle ou un groupe alkyl(en C₁ - C₆)sulfonyle ;
R³ représente un atome d'hydrogène, un atome d'halogène ou un groupe alkyle en C₁ - C₆ ;
R⁴ représente un atome d'hydrogène ou un atome d'halogène ;
R⁵ représente un groupe cyano, un atome d'halogène, un groupe alkyle en C₁ - C₆, un groupe halogénalkyle en C₁ - C₆, un groupe alcoxy en C₁ - C₆ ou un groupe halogénalcoxy en C₁ - C₆ ;
R⁶ représente un atome d'hydrogène, un groupe cycloalkyle en C₃ - C₇ ou un groupe hétérocyclyle saturé de 3 à 7 membres, contenant un ou plusieurs atomes d'oxygène et/ou un ou plusieurs atomes de soufre, chaque noyau cycloalkyle et chaque noyau hétérocyclyle pouvant contenir un chaînon carbonyle ou un chaînon thiocarbonyle ;
et dans lequel chaque noyau cycloalkyle et chaque noyau hétérocyclyle peut être non substitué ou peut porter de un à quatre substituants respectivement choisi parmi le groupe constitué par un groupe cyano, un groupe nitro, un groupe amino, un groupe hydroxyle, un atome d'halogène, un groupe alkyle en C₁ - C₄, un groupe halogénalkyle en C₁ - C₄, un groupe cyanoalkyle en C₁ - C₄, un groupe hydroxyalkyle en C₁ - C₄, un groupe aminoalkyle en C₁ - C₄, un groupe alcoxy en C₁ - C₄, un groupe halogénalcoxy en C₁ - C₄, un groupe alkyl(en C₁ - C₄)thio, un groupe halogénalkyl(en C₁ - C₄)thio, un groupe alkyl(en C₁ - C₄)-sulfinyle, un groupe alkyl(en C₁ - C₄)sulfonyle, un groupe halogénalkyl(en C₁ - C₄)sulfonyle, un groupe alcoxy(en C₁ - C₄)-carbonyle, un groupe alkyl(en C₁ - C₄) carbonyle, un groupe halogénalkyl(en C₁ - C₄)carbonyle, un groupe alkyl(en C₁ - C₄)-carbonyloxy, un groupe halogénalkyl(en C₁ - C₄)carbonyloxy, un groupe dialkyl(en C₁ - C₄)amino, un groupe alcényle en C₃ - C₆, un groupe alcynyle en C₃ - C₆, un groupe alcényl(en C₃ - C₄)oxy, un groupe alcényl(en C₃ - C₄)thio, un groupe alcynyl(en C₃ - C₄)-oxy et un groupe alcynyl(en C₃ - C₄)thio,
avec cette réserve que le radical R⁶ ne représente un atome d'hydrogène que lorsque Z représente une liaison chimique,
ainsi que les sels des composés I utilisables en agriculture.

2. 3-[benzoxazol-7-yl]-1H-pyrimidine-2,4-diones répondant à la formule I selon la revendication 1, dans lesquelles
X représente un atome d'oxygène ;
Z représente une liaison chimique, un groupe alkylène en C₁ - C₄, un atome d'oxygène ou un atome de soufre ;
R¹ représente un atome d'hydrogène, un groupe amino ou un groupe alkyle en C₁ - C₆ ;
R² représente un atome d'hydrogène, un atome d'halogène, un groupe alkyle en C₁ - C₆, un groupe halogénalkyle en C₁ - C₆ ou un groupe alkyl(en C₁ - C₆)sulfonyle ;
R³ représente un atome d'hydrogène ;
R⁴ représente un atome d'hydrogène, un atome de fluor ou un atome de chlore, et
R⁵ représente un groupe cyano ou un atome d'halogène.

3. Utilisation des 3-[benzoxazol-7-yl]-1H-pyrimidine-2,4-diones répondant à la formule I, et de leurs sels utilisables en agriculture, selon la revendication 1, à titre d'herbicides.

4. Agent herbicide, contenant une quantité efficace du point de vue herbicide d'au moins une 3-[benzoxazol-7-yl]-1H-pyrimidine-2,4-dione répondant à la formule I ou d'un de ses sels utilisables en agriculture, selon la revendication 1, et au moins une substance de support liquide et/ou solide, ainsi que, le cas échéant, au moins une substance tensioactive.

5. Procédé pour la préparation d'agents à activité herbicide, **caractérisé en ce qu'**on mélange une quantité efficace du point de vue herbicide d'au moins une 3-[benzoxazol-7-yl]-1H-pyrimidine-2,4-dione répondant à la formule I ou d'un de ses sels utilisables en agriculture, selon la revendication 1, avec au moins une substance de support liquide et/ou solide inerte, ainsi que, le cas échéant, avec au moins une substance tensioactive.

6. Procédé pour lutter contre la croissance de plantes non désirées, **caractérisé en ce qu'**on laisse agir une quantité efficace du point de vue herbicide d'au moins une 3-[benzoxazol-7-yl]-1H-pyrimidine-2,4-dione répondant à la formule I ou d'un de ses sels utilisables en agriculture, selon la revendication 1, sur des plantes, sur leur biotope ou encore sur des semences.

7. Procédé pour la préparation de 3-[benzoxazol-7-yl]-1H-pyrimidine-2,4-diones répondant à la formule selon la revendication 1, **caractérisé en ce qu'**on soumet des aminophénols respectivement des aminothiophénols répondant à la formule VII 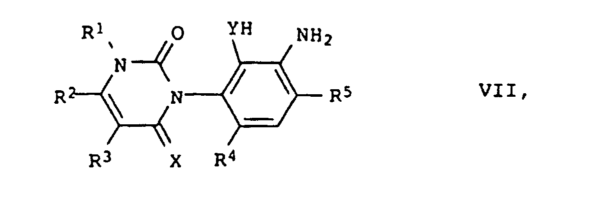 dans laquelle les variables X et R¹ à R⁵ ont les significations indiquées à la revendication 1, à une condensation avec des dérivés d'acides carboxyliques, respectivement de l'acide carbonique.
